# EUROPEAN PATENT APPLICATION

(11) **EP 2 949 648 A1**
(43) Date of publication of application: **02.12.2015**
(21) Application number: 14170656.4
(22) Date of filing: 30.05.2014
(51) Int. Cl.: C07D 241/04, C07D 263/22, C07D 265/30, C07D 211/14, A61K 31/549, A61K 31/5375, A61P 35/00

(54) **Cyclopropylamine derivatives as histone demethylase inhibitors**

(71) Applicant: IEO - Istituto Europeo di Oncologia Srl, 20121 Milano (IT)
(72) Inventor: Vianello, Paola, 20149 Milano (MI) (IT); Varasi, Mario, 20142 Milano (MI) (IT); Mercurio, Ciro, 20025 Legnano (IT); Cappa, Anna, 62039 Visso (IT); Meroni, Giuseppe, 20142 Milano (MI) (IT); Villa, Manuela, 22040 Lurago d'Erba (IT); Mai, Antonello, 00137 Roma (RM) (IT); Valente, Sergio, 03043 Cassino (FR) (IT)
(74) Representative: Cooley (UK) LLP

(57) **Abstract**

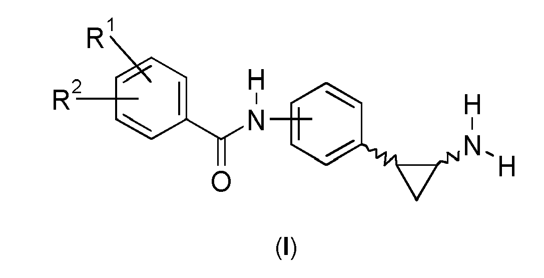

The present invention relates to cyclopropyl derivatives of general formula (I), wherein **R¹,** and **R²** are as defined in the specification. The present application also relates to pharmaceutical compositions containing such compounds and to their use in therapy.

## Description

### Field of the Invention

The present invention relates to cyclopropyl derivatives, pharmaceutical compositions containing such compounds and to their use in therapy.

### Background of the invention

Alterations in the structural and functional states of chromatin, mainly determined by post-translational modification of histone components, are involved in the pathogenesis of a variety of diseases. The enzymatic processes governing these post-translational modifications on the nucleosomes have become potential targets for the so-called epigenetic therapies (Portela, A. et al. Nat. Biotechnol. 2010, 28, 1057-1068).

The discovery of an increasing number of histone lysine demethylases has highlighted the dynamic nature of the regulation of histone methylation, a key chromatin modification that is involved in eukaryotic genome and gene regulation. Histone lysine demethylases represent attractive targets for epigenetic drugs, since their expression and/or activities are often misregulated in cancer (Varier, R. A. et al. Biochim. Biophys. Acta. 2011, 1815, 75-89). A lysine can be mono-, di-, and tri-methylated and each modification, even on the same amino acid, can exert different biological effects.

Histone lysine demethylases exert their activity through two different type of mechanism (Anand, R. et al. J. Biol. Chem. 2007, 282, 35425-35429; Metzger, E. et al. Nat. Struct. Mol. Biol. 2007, 14, 252-254). While the Jumonji domain-containing histone demethylases, which are iron and 2-oxoglutarate dependent oxygenases, act on mono-, di- and trimethylated lysines, the flavin-dependent (FAD) histone demethylases catalyse the cleavage of mono and dimethylated lysine residues. Currently, two FAD dependent demethylases have been identified: LSD1, also known as KDM1A, and LSD2, also known as KDM1B. (Culhane, J. C. et al.. Curr Opin Chem Biol 2007, 11, 561-568, Ciccone, D. N. et al.. Nature 2009, 461, 415-418).

KDM1A is a constituent in several chromatin-remodeling complexes and is often associated with the co-repressor protein CoREST. KDM1A specifically removes the methyl groups from both mono- and di-methyl Lys4 of histone H3, which is a well-characterized gene activation mark.

KDM1A represents an interesting target for epigenetic drugs as supported by data related to its over-expression in solid and hematological tumors (Schulte, J. H. et al. Cancer Res. 2009, 69, 2065-2071; Lim, S. et al. Carcinogenesis 2010, 31, 512-520; Hayami, S. et al. Int. J. Cancer 2011, 128, 574-586; Schildhaus, H. U. et al. Hum. Pathol. 2011, 42, 1667-1675; Bennani-Baiti, I. M. et al. Hum. Pathol. 2012, 43, 1300-1307). Its over-expression correlates to tumor recurrence in prostate cancer (Kahl, P. et al. Cancer Res. 2006, 66, 11341-11347), and has a role in various differentiation processes, such as adipogenesis (Musri, M. M. et al. J. Biol. Chem. 2010, 285, 30034-30041), muscle skeletal differentiation (Choi, J. et al. Biochem. Biophys. Res. Commun. 2010, 401, 327-332), and hematopoiesis (Hu, X. et al. Proc. Natl. Acad. Sci. USA 2009, 106, 10141-10146; Li, Y. et al. Oncogene. 2012, 31, 5007-18). KDM1A is further involved in the regulation of cellular energy expenditure (Hino S. Et al. Nat Commun. 2012, doi: 10.1038/ncomms1755), in the control of checkpoints of viral gene expression in productive and latent infections (Roizman, B. J. Virol. 2011, 85, 7474-7482) and more specifically in the control of herpes virus infection (Gu, H. J. Virol. 2009, 83, 4376-4385) and HIV transcription (Sakane, N. et al. PLoS Pathog. 2011, 7(8):e1002184). The role of KDM1A in the regulation of neural stem cell proliferation (Sun, G. et al. Mol. Cell Biol. 2010, 30, 1997-2005) as well as in the control of neuritis morphogenesis (Zibetti, C. et al. J. Neurosci. 2010, 30, 2521-2532) suggests its possible involvement in neurodegenerative diseases.

Furthermore, there are evidences of the relevance of KDM1A in the control of other important cellular processes, such as DNA methylation (Wang, J. et al. Nat. Genet. 2009, 41(1):125-129), cell proliferation (Scoumanne, A. et al. J. Biol. Chem. 2007, 282, 15471-15475; Cho, H. S. et al. Cancer Res. 2011, 71, 655-660), epithelial mesenchimal transition (Lin, T. et al. Oncogene. 2010, 29, 4896-4904) and chromosome segregation (Lv, S. et al. Eur. J. Cell Biol. 2010, 89, 557-563). Moreover, it was found that KDM1A inhibitors were able to reactivate silenced tumor suppressor genes (Huang, Y. et al. Proc. Natl. Acad. Sci. U S A. 2007, 104, 8023-8028; Huang, Y. et al. Clin. Cancer Res. 2009, 15, 7217-7228), to target selectively cancer cells with pluripotent stem cell properties (Wang, J. et al. Cancer Res. 2011, 71, 7238-7249), as well as to reactivate the all-trans-retinoic acid differentiation pathway in acute myeloid leukemia (Schenk, T. et al. Nat Med. 2012, 18, 605-611).

The more recently discovered demethylase KDM1B (LSD2) displays -similarly to KDM1A- specificity for mono- and di-methylated Lys4 of histone H3. KDM1 B, differently from KDM1A, does not bind CoREST and it has not been found up to now in any of KDM1A-containing protein complex (Karytinos, A. et al. J. Biol. Chem. 2009, 284, 17775-17782). On the contrary, KDM1B forms active complexes with euchromatic histone methyltransferases G9a and NSD3 as well as with cellular factors involved in transcription elongation. KDM1B has been reported to have a role as regulator of transcription elongation rather than that of a transcriptional repressor as proposed for KDM1A (Fang, R. et al. Mol. Cell 2010, 39, 222-233).

KDM1A and KDM1B are both flavo amino oxidase dependent proteins sharing a FAD coenzyme-binding motif, a SWIRM domain and an amine oxidase domain, all of which are integral to the enzymatic activity of KDM1 family members. Moreover, both KDM1A and KDM1B show a structural similarity with the monoamine oxidases MAO-A and MAO-B.

Indeed, tranylcypromine, a MAO inhibitor used as antidepressant agent, was found to be also able to inhibit LSD1. The compound acts as an irreversible inhibitor forming a covalent adduct with the FAD cofactor. (Lee, M. G. et al. Chem. Biol. 2006, 13, 563; Schmidt, D. M. Z. et al. Biochemistry 2007, 46, 4408).

The synthesis of tranylcypromine analogs and their LSD1 inhibitory activity has been described in Bioorg. Med. Chem. Lett. 2008, 18, 3047-3051, in Bioorg. Med. Chem. 2011, 19, 3709-3716 in J. Am. Chem. Soc 2011, 132, 6827-6833. Further arylcyclopropylamine and heteroarylcyclopropylamine derivatives as LSD1, MAO-A and/or MAO-B enzyme inhibitors are disclosed in US2010/324147, in WO2012/045883 and in WO2013/022047.

WO2011/131576 discloses tranylcyclopropylamine derivatives of the general formula 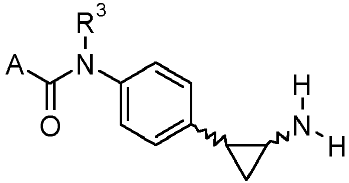 wherein
**A** is R or CH(R₁)-NH-CO-R₂; **R** and **R₂** are selected from: alkyl, alkenyl, alkynyl, cycloalkyl, aryl, heteroaryl, heterocycloalkyl, cycloalkylalkyloxy, arylalkyloxy, heteroarylalkyloxy, heterocycloalkylalkyloxy, cycloalkylalkyl, arylalkyl, heteroarylalkyl, heterocycloalkylalkyl, cycloalkylalkylamino, arylalkylamino, heteroarylalkylamino, heterocycloalkylalkylamino; **R₁** is selected from: alkyl, alkenyl, alkynyl, cycloalkyl, aryl, heteroaryl, heterocycloalkyl, cycloalkylalkyl, arylalkyl, heteroarylalkyl, heterocycloalkylalkyl; and **R₃** is H, lower alkyl.

However, there is still the need for compounds endowed with inhibitory activity on KDM1A and/or KDM1B enzyme activity with a low inhibitory activity on MAOA and/or MAOB enzymes.

MAOs are well known targets for the treatment of diseases of the central nervous system, such as depression or Parkinson's disease. However, inhibition of the MAOs are associated with side effects, among them tyramine-induced hypertensive crisis or the serotonin syndrome, which occurs in situation of concomintant use of MAO inhibitors and other serotoninergic drugs. (Wimbiscus, M. et al. Cleve. Clin. J. Med., 2010, 859-882; lqbal, M. M. Ann. Clin. Psychiatry, 2012, 24, 310-318).

### Summary of the Invention

The present invention relates to substituted cyclopropylamine derivatives having highly potent inhibitory activities of the KDM1A enzyme and/or of the KDM1B enzyme and low inhibitory activity of monoamine oxidases (MAOs), useful in the prevention or therapy of diseases and conditions associated with the activity of the histone demethylases.

According to the present invention there are provided compounds, endowed with a potent KDM1A (LSD1) inhibitory activity, of general formula (**I**) 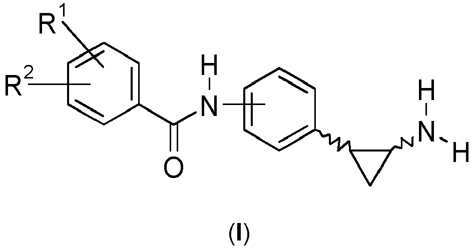 wherein:
- **R¹**: is heterocyclyl or heterocyclyl substituted by oxo, wherein the heterocyclyl is unsubstituted or substituted by one or more C₁-C₆ alkyl;
- **R²**: is hydrogen; halogen; C₁-C₆ alkyl; C₁-C₆ alkoxy; C₁-C₆ haloalkyl; or C₁-C₆ haloalkoxy; or benzyloxycarbonylamino;
or stereoisomers or pharmaceutically acceptable salts thereof.

Preferably **R¹** is pyrrolidinyl, piperidinyl, piperazinyl, tetrahydrofuranyl, tetrahydropyranyl, morpholinyl, thiomorpholinyl, tetrahydroquinolinyl, tetrahydroisoquinolinyl, tetrahydroquinoxalinyl, benzodioxolyl, 2,3-dihydro-benzodioxinyl, benzoxazolyl, azepinyl, diazapinyl or 2-oxooxazolidinyl.

More preferably **R¹** is 4-methylpiperazin-1-yl, 1-methylpiperidin-4-yl, piperidin-1-yl, or 2-oxooxazolidin-3-yl.

In a further embodiment **R²** is selected from the group consisting of hydrogen or benzyloxycarbonylami no.

Preferred compounds are selected from:
*N*-[4-[*trans*-2-aminocyclopropyl]phenyl]-4-(4-methylpiperazin-1-yl)benzamide;
*N*-[4-[*trans*-2-aminocyclopropyl]phenyl]-4-(1-methyl-4-piperidyl)benzamide;
*N*-[4-[*trans*-2-aminocyclopropyl]phenyl]-3-(2-oxooxazolidin-3-yl)benzamide;
*N*-[4-[*trans*-2-aminocyclopropyl]phenyl]-4-morpholino-benzamide;
*N*-[4-[*trans*-2-aminocyclopropyl]phenyl]-4-(2-oxooxazolidin-3-yl)benzamide;
benzyl *N*-[5-[[4-[(*trans*-2-aminocyclopropyl]phenyl]carbamoyl]-2-(4-methylpiperazin-1-yl)phenyl]carbamate;
benzyl *N*-[4-[[4-[*trans*-2-aminocyclopropyl]phenyl]carbamoyl]-2-(4-methylpiperazin-1-yl)phenyl]carbamate;
benzyl *N*-[5-[[4-[*trans*-2-aminocyclopropyl]phenyl]carbamoyl]-2-(1-piperidyl)phenyl]carbamate;
benzyl *N*-[5-[[4-[*trans*-2-aminocyclopropyl]phenyl]carbamoyl]-2-morpholinophenyl]carbamate
or stereoisomers or pharmaceutically acceptable salts thereof.

In a further embodiment the compounds of the invention are for use as KDM1 inhibitor. In a further embodiment the compounds of the invention are for use as a medicament. In a further embodiment the compounds of the invention are for use in the treatment and/or prevention of cancer, infectious diseases or a disease characterized by aberration of cellular energy metabolism, such as obesity.

Preferably, the cancer is leukemia, non-small cell lung cancer, hepatocellular carcinoma, or glioblastomas. Still preferably the glioblastomas are giant cell glioblastoma or gliosarcoma.

It is a further embodiment of the invention a pharmaceutical composition comprising a compound as defined above, together with a pharmaceutically acceptable excipient and/or diluent.

Preferably, the pharmaceutical composition further comprises at least one therapeutic agent, preferably selected from the group consisting of: histone deacetylase inhibitors, retinoid receptor modulators, anti-proliferative/antineoplastic agents, cytostatic agents, agents which inhibit cancer cell invasion, inhibitors of growth factor function, antiangiogenic agents, cell cycle inhibitors, proteasome inhibitors, HSP90 inhibitors, selective COX-2 inhibitors or a chemotherapeutic agent.

In a further embodiment the pharmaceutical composition is in the form of tablets, capsules, oral preparations, powders, granules, pills, injectable or infusible liquid, solutions, suspensions, emulsions, suppositories, ointments, creams, lotions, gels, pastes, transdermal delivery devices.

It is a further embodiment of the invention a process for obtaining a compound of formula **(I)** as defined above, the process comprising the preparation of compounds of formula **A2** by reaction of a compound of formula **A1** with the a suitable azide and in presence of a base, the reaction of a compound of formula **A2** with an amide **A3** and CuI in presence of a base to obtain a compound of formula **A4,** and the deprotection of a compound of formula **A4** to obtain a compound of formula **(I),** as represented in **Scheme A** below: 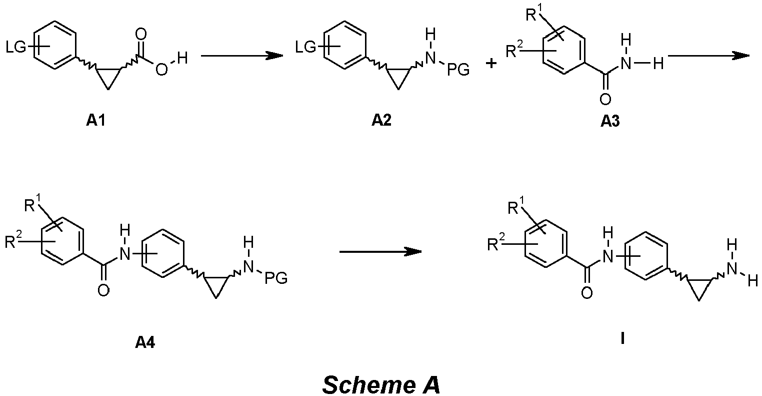 wherein **R¹** and **R²** are as defined herein above, **PG** is a protecting group, and **LG** is a leaving group.

Preferably **PG** is carboxybenzyl, *tert*-butyloxycarbonyl (BOC), or 9-fluorenylmethyloxycarbonyl.

It is a further embodiment of the invention a process for obtaining a compound of formula **(I)** as defined above, the process comprising the preparation of compounds of formula **B3** by reaction of a compound of formula **B1** with compound of formula **B2** and in presence of a condensating agent, and the deprotection of a compound of formula **B3** to obtain a compound of formula **(I),** as represented in **Scheme B** below: 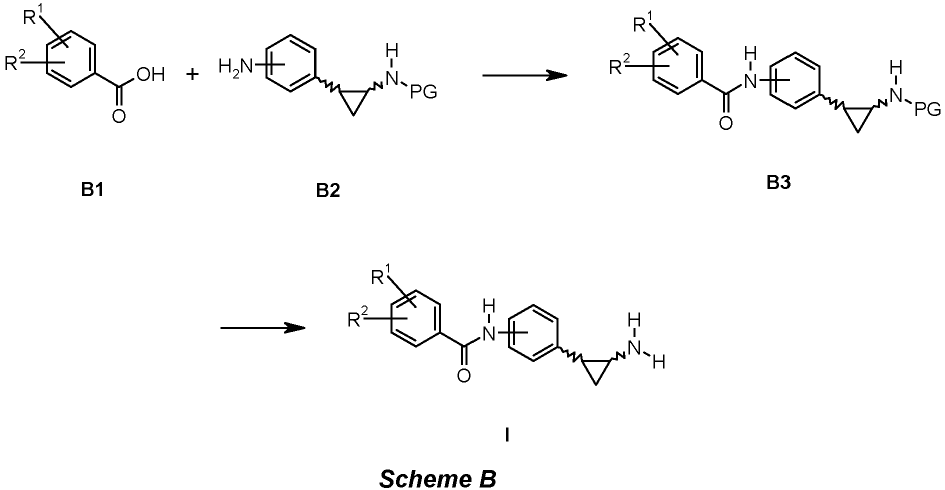 wherein **R¹**, **R²** and **PG** are as defined herein above.

In a preferred embodiment **R¹** is morpholin, 4-methylpiperazin-1-yl, 1-methylpiperidin-4-yl, piperidin-1-yl, or 2-oxooxazolidin-3-yl.

It is a further embodiment of the invention a kit comprising a compound as defined above and at least one therapeutic agent, preferably selected from the group consisting of: histone deacetylase inhibitors, retinoid receptor modulators, anti proliferative/antineoplastic agents, cytostatic agents, agents which inhibit cancer cell invasion, inhibitors of growth factor function, antiangiogenic agents, cell cycle inhibitors, proteasome inhibitors, HSP90 inhibitors, Selective COX-2 inhibitors or a chemotherapeutic agent for use in the treatment and/or prevention of cancer, infectious diseases or a disease characterized by aberration of cellular energy metabolism, such as obesity. Optionally, the compound of the invention and the at least one therapeutic agent are in separated containers.

In the present invention, "Heterocyclyl" represents a mono or bicyclic saturated or partially saturated non-aromatic ring system of, respectively, 4 to 12 members, which contains one, two, or three heteroatoms selected from nitrogen, oxygen, and sulphur and three to eleven carbon atoms. Examples of such heterocycles include, but are not limited to: pyrrolidinyl, piperidinyl, piperazinyl, tetrahydrofuranyl, tetrahydropyranyl, morpholinyl, thiomorpholinyl, tetrahydroquinolinyl, tetrahydroisoquinolinyl, tetrahydroquinoxalinyl, benzodioxolyl, 2,3-dihydro-benzodioxinyl, benzoxazolyl, azepinyl, and diazapinyl. "Heterocyclyl substituted by oxo" represents a mono or bicyclic saturated or partially saturated non-aromatic ring system of, respectively, 4 to 12 members, which contains one, two, or three heteroatoms selected from nitrogen, oxygen, and sulphur, and which is substituted by an oxo group. Examples include, but are not limited to 2-oxooxazolidin-3-yl.

The term "halogen" refers to fluoro, chloro, bromo, or iodo.

The term "C₁-C₆ alkyl" refers to a straight or branched hydrocarbon chain radical, consisting solely of carbon and hydrogen atoms, having from one to six carbon atoms. Suitable examples of C₁-C₆ alkyl include methyl, ethyl, n-propyl, isopropyl, butyl, *tert-*butyl, pentyl, and hexyl.

The term "C₁-C₆ alkoxy" refers to a straight or branched O-C₁-C₆ alkyl, where alkyl is as defined herein. The "C₁-C₆ alkoxy" group is preferably a linear or branched C₁-C₄ alkoxy group, more preferably a C₁-C₂ alkoxy group.

The term "C₁-C₆ haloalkyl" refers to a straight or branched hydrocarbon chain radical, which is substituted by one or more halogen atoms and having from one to six carbon atoms. The "C₁-C₆ haloalkyl" group is preferably a linear or branched C₁-C₄ haloalkyl group, more preferably a C₁-C₂ haloalkyl group, being in particular CF₃.

The term "C₁-C₆ haloalkoxy" refers to a straight or branched O-C₁-C₆ haloalkyl, where haloalkyl is as defined herein. The "C₁-C₆ haloalkoxy" group is preferably a linear or branched C₁-C₄ haloalkoxy group, more preferably a C₁-C₂ haloalkoxy group, being in particular OCF₃, OCHF₂ or OCH₂F.

Pharmaceutically acceptable salts comprise conventional non-toxic salts obtained by salification of a compound of formula (I) with inorganic acids (e.g. hydrochloric, hydrobromic, sulphuric, or phosphoric acids), or with organic acids (e.g. acetic, propionic, succinic, benzoic, sulfanilic, 2-acetoxy-benzoic, cinnamic, mandelic, salicylic, glycolic, lactic, oxalic, malic, maleic, malonic, fumaric, tartaric, citric, p-toluenesulfonic, methanesulfonic, ethanesulfonic, or naphthalensulfonic acids). For reviews on suitable pharmaceutical salts see Berge S. M. et al., J. Pharm. Sci. 1977, 66, 1-19; Gould P. L. Int. J. Pharm 1986, 33, 201-217; and Bighley et al. Encyclopedia of Pharmaceutical Technology, Marcel Dekker Inc, New York 1996, Volume 13, page 453-497. Other salts, which are not pharmaceutically acceptable, for example the trifluoroacetate salt, may be useful in the preparation of compounds of this invention and these form a further aspect of the invention. The invention includes within its scope all possible stoichiometric and non-stoichiometric forms of the salts of the compounds of formula (I).

In addition, the compounds of formula (I) may exist in unsolvated as well as in solvated forms with pharmaceutically acceptable solvents such as water, EtOH and the like. Certain compounds of formula (I) may exist in stereoisomeric forms (e.g. they may contain one or more asymmetric carbon atoms). The individual stereoisomers (enantiomers and diastereomers) and mixtures of these are included within the scope of the present invention. The present invention also covers the individual isomers of the compounds represented by formula (I) as mixtures with isomers thereof in which one or more chiral centres are inverted.

The invention also includes all suitable isotopic variations of a compound of the invention. Examples of isotopes that can be incorporated into compounds of the invention include isotopes such as ²H, ³H, ¹³C ¹⁴C, ¹⁵N, ¹⁷O, ¹⁸O, ³¹P, ³²P, ³⁵S, ¹⁸F and ³⁶Cl, respectively. Certain isotopic variations of the invention, for example, those in which a radioactive isotope such as ³H or ¹⁴C is incorporated, are useful in drug and/or substrate tissue distribution studies. Further, substitution with isotopes such as deuterium ²H, may afford certain therapeutic advantages resulting from greater metabolic stability. Isotopic variations of the compounds of the invention can generally be prepared by conventional procedures such as by the illustrative methods or by the preparations described in the examples hereafter using appropriate isotopic variations of suitable reagents.

Compounds of general formula **(I)** may be prepared according to **Scheme A:** 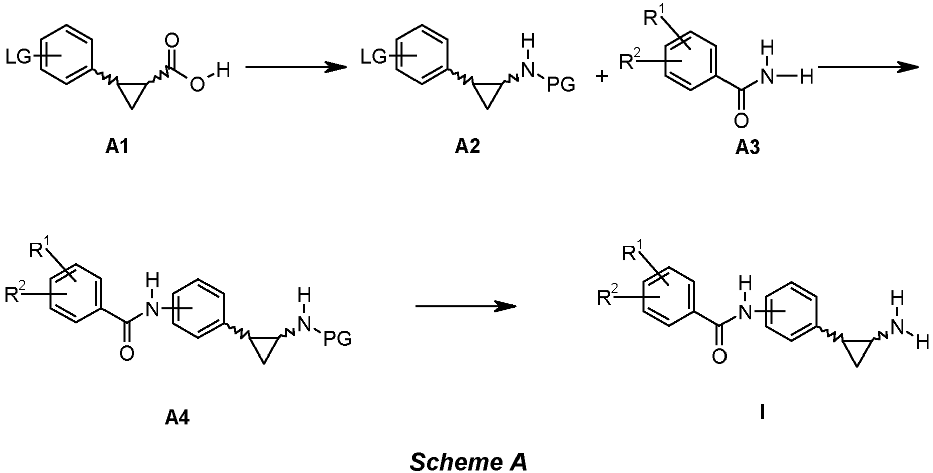 wherein **R¹** and **R²** are as defined above for formula **(I), PG** is a protecting group chosen among those known in the art, for example carboxybenzyl, *tert*-butyloxycarbonyl (BOC), 9-fluorenylmethyloxycarbonyl, etc. and **LG** is a leaving group, for example bromide, iodide or chloride.

Compounds of formula **A1** and **A3** are known compounds or may be prepared by known methods.

The carboxylic acid of formula **A1** may be converted into the protected amine of formula **A2** by reaction with a suitable azide, such as diphenyl phosphoryl azide, in the presence of a suitable base (e.g. triethylamine) and in a suitable solvent such as *tert*-butanol at a temperature ranging from room temperature to the boiling point of the solvent.

A compound of formula **A4,** may be prepared according to the Ullmann type reaction by reacting a compound of formula **A2** and a compound of formula **A3** with CuI in presence of a suitable base, such as Cs₂CO₃, K₂CO₃, triethylamine, *N,N*'-dimethylethane-1,2-diamine, *N,N*'-dimethylcyclohexane-1,2-diamine or 2-aminoethanol, in a suitable solvent, for example in dimethylacetamide, tetrahydrofuran or dioxane, at a temperature ranging from room temperature to the boiling point of the solvent.

A compound of formula **A4** may be deprotected to obtain a compound of formula **(I)** according to known methods, e.g. by treatment of a BOC derivative with HCl or TFA (trifluoroacetic acid) in a suitable solvent such as dioxane, Et₂O or dichloromethane, at a temperature ranging from 0°C to room temperature.

Alternatively, compounds of general formula **(I)** may be prepared according to **Scheme B:** 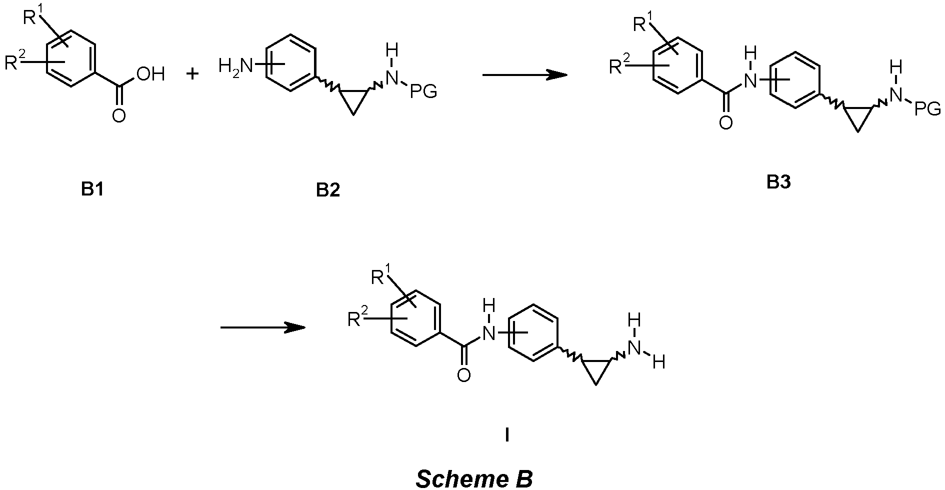 wherein **R¹**, **R²** and **PG** are as defined above.

The reaction of a compound of formula **B1** with a compound of formula **B2** can be carried out with condensating agents such as PyBOP (benzotriazol-1-yl-oxytripyrrolidinophosphonium hexafluorophosphate) or EDC (1-(3-dimethylaminopropyl)-3-ethylcarbodiimide), optionally in the presence of a suitable base (e.g. triethylamine or di-isopropylethylamine) in a suitable solvent (e.g. tetrahydrofuran, dichloromethane or DMF). Generally an activator of the condensation reaction, such as HOBt (1-hydroxybenzotriazole) or HOAt (1-hydroxy-7-aza-benzotriazole), can be added to the reaction mixture. The reaction can be carried out at room temperature for a period lasting between about 2 and 24 h. A compound of formula **B3** may be deprotected to obtain a compound of formula **(I)** according to known methods, e.g. by treatment of a BOC derivative with HCl or TFA (trifluoroacetic acid) in a suitable solvent such as dioxane, Et₂O or dichloromethane, at a temperature ranging from 0°C to room temperature.

Compounds of formula **B2** are known compounds (J. Am. Chem. Soc 2010, 132, 6827-6833, GB2404658) or may be prepared by known methods.

Compounds of formula **B1** are known compounds or may be prepared by known methods. In case, wherein **R¹** is morpholin, 4-methylpiperazin-1-yl, or piperidin-1-yl and **R²** is benzyloxycarbonylamino, compounds of formula **B1** can be prepared according to the following **Scheme C:** 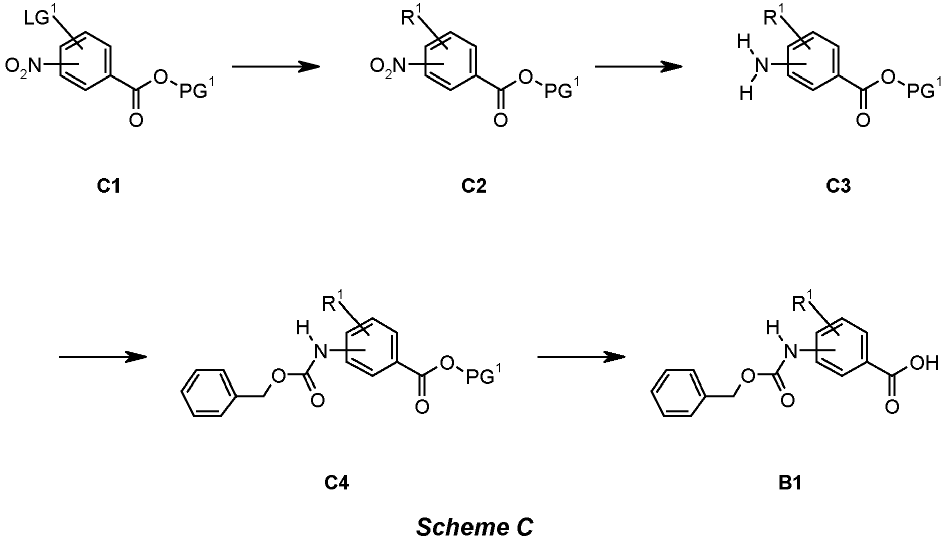 wherein **PG¹** is a protecting group chosen among those known in the art, for example methyl, *tert*-butyl, etc., and **LG¹** is a leaving group, for example fluoride or chloride. Compounds of formula **C1** are known compounds or may be prepared by known methods.

Compounds of formula **C1** can be converted into compound of formula **C2** by reaction with morpholine, 4-methylpiperazine, or piperidine in presence of a base (e.g. K₂CO₃) in a suitable solvent (e.g. tetrahydrofuran, dichloromethane or DMF). Reduction of the nitro group to the amine in compound of formula **C3** can be achieved by conventional catalytic hydrogenation, e.g. in a Parr or H-Cube apparatus using a palladium or platinum catalyst. Compound of formula **C3** was then converted into a compound of formula **C4** by reaction with benzyl chloroformate in presence of a suitable base, such as triethylamine, in a suitable solvent, for example in tetrahydrofuran or dioxane, at a temperature ranging from 0°C to room temperature. A compound of formula **C4** may be deprotected to obtain a compound of formula **C4** according to known methods, e.g. by treatment of a *tert*-butyl derivative with HCl or TFA (trifluoroacetic acid) in a suitable solvent such as dioxane, THF, Et₂O or dichloromethane, at a temperature ranging from 0°C to room temperature.

In the case it is necessary to protect a chemical group of a compound of the present invention and/or an intermediate thereof, before carrying out one of the aforedescribed reactions, said chemical group can be protected and deprotected according to known methods. A thorough discussion for protection/deprotection steps is provided for example in Greene and Wuts (Greene, T.W.; Wuts, P.G.M. "Protective Groups in Organic Synthesis", John Wiley & Sons Inc., 2006) or in Kocienski (Kocienski, P.J. "Protecting Groups", George Thieme Verlag, 2005).

Salification of the compounds of formula **(I),** and preparation of compounds of formula **(I),** free of their salts, can be carried out by known conventional methods.

In view of the above described mechanisms of action, the compounds of the present invention are useful in the prevention or treatment of tumor type diseases, including but not limited to: acute and chronic myeloid leukaemia, acute and chronic lymphoblastic leukaemia, myelodysplastic syndromes, multiple myeloma, Hodgkin's disease, non-Hodgkin's lymphomas, cutaneous and peripheral T-cell lymphoma, adult T-cell leukemia, large B-cell lymphoma; mammary tumors; pulmonary tumors and pleural mesotheliomas, adenocarcinoma, non-small lung cancer, small-cell lung cancer; skin tumors including basal cell carcinomas (basaliomas), melanomas, squamous cell carcinoma, Kaposi's sarcoma, keratocanthomas; osteosarcomas, fibrosarcomas, rhabdomyosarcomas, neuroblastomas, glioblastomas, cerebral tumors, head and neck cancer, testicular and ovarian tumors, cervical carcinoma, endometrial and prostate tumors (for example advanced prostate cancer), thyroid carcinomas (for example tyroid follicular cancer), colon cancers (for example colon adenocarcinoma, colon adenoma), gastric tumors and gastrointestinal adenocarcinomas, hepatocellular carcinomas, pancreatic carcinomas (for example exocrine pancreatic carcinoma), renal tumors, teratocarcinomas and embryonic carcinomas.

The compounds of the invention are also useful in the prevention or treatment of infections, including, but not limited to, infections caused by protozoa, fungi, phytotoxic agents, viruses and parasites, for example HIV or herpes virus infections.

Considering the role of KDM1A in the regulation of cellular energy expenditure in the adypocites as well as the direct relation between KDM1A function and its target genes in adipose tissue of high fat diet mice, the compounds of the invention are also useful in the prevention or treatment of diseases characterized by aberration of cellular energy metabolism, such as obesity (Hino S. Et al. Nat Commun. 2012, doi: 10.1038/ncomms1755).

The compounds of formula **(I)** can also be used in combination with additional agents, in particular anti-tumor and differentiating agents, either by separate administrations, or by including the two active principles in the same pharmaceutical formulation. Non-exhaustive examples of suitable additional agents include:
a) histone deacetylase inhibitors (for example, but not limited to SAHA, PXD101, JNJ-26481585, SB939, ITF-2357, LBH589, PCI-24781, valproic acid, butyric acid, MS-275, MGCD0103 and FK-228);
b) retinoid receptor modulators such as 13-*cis*-retinoic acid, 9-*cis*-retinoic acid, bexarotene, alitretinoin, or tretinoin; vitamin D;
c) anti proliferative/antineoplastic drugs and combinations thereof, as used in medical oncology, such as alkylating agents (for example platin derivatives like *cis-*platin, carboplatin, oxaliplatin, lobaplatin, satraplatin, nedaplatin, heptaplatin; nitrogen mustard such as chlorambucil, melphalan, chlormethine, cyclophosphamide, ifosfamide, trofosfamide, uramustine, bendamustine, estramustine; busulphan, temozolomide or nitrosoureas); antimetabolites (for example antifolates such as aminopterin, methotrexate, pemetrexed, raltitrexed); purines such as cladribine, clofarabine, fludarabine, mercaptopurine, pentostatin, thioguanine; pyrimidines like capecitabine, cytarabine, fluorouracil, floxuridine, gemcitabine; azacitidine, decitabine; cytosine arabinoside or hydroxyurea; antitumour antibiotics (for example anthracyclines like aclarubicin, amrubicin, daunomycin, doxorubicin, epirubicin, idarabicin, valrubicin, zorubicine; mitoxantrone; or antibiotics from streptomyces like actinomycin, bleomycin, mitomycin, or plicamycin); antimitotic agents (for example vinca alkaloids like vincristine, vinblastine, vindesine or vinorelbine; taxoids like docetaxel, paclitaxel or tesetaxel; epothilones like ixabepilone) and topoisomerase inhibitors (for example epipodophyllotoxins like etoposide and teniposide; amsacrine, camptothecin, irinotecan, rubitecan, and topotecan);
d) cytostatic agents such as antioestrogens (for example tamoxifen, toremifene, raloxifene, droloxifene and idoxifene), oestrogen receptor down regulators (for example fulvestrant), antiandrogens (for example bicalutamide, flutamide, nilutamide, liarozole or cyproterone acetate), LHRH antagonists or LHRH agonists (for example goserelin, leuprorelin or buserelin), progestogens (for example megestrol acetate), aromatase inhibitors (for example as anastrozole, letrozole, vorazole and exemestane) and inhibitors of 5-alpha-reductase such as finasteride;
e) agents which inhibit cancer cell invasion (for example metalloproteinase inhibitors and inhibitors of urokinase plasminogen activator receptor function);
f) inhibitors of growth factor function, for example growth factor antibodies, growth factor receptor antibodies (for example the anti-erbb2 antibody trastuzumab, the anti-erbbl antibody cetuximab and panitumumab, the anti IGF1R antibody figitumumab), farnesyl transferase inhibitors, MEK inhibitors, tyrosine kinase inhibitors and serine/threonine kinase inhibitors, for example enzastaurin, dasatinib, erlotinib, gefitinib, imatinib, lapatinib, nilotinib, sorafenib, sunitinib, everolimus, sirolimus or temsirolimus;
g) antiangiogenic agents such as those which inhibit the effects of vascular endothelial growth factor, for example the anti-vascular endothelial cell growth factor antibody bevacizumab, lenalidomide or thalidomide;
h) cell cycle inhibitors including for example CDK inhibitors (for example but not limited to flavopiridol, roscovitine) and other inhibitors of cell cycle checkpoints; inhibitors of aurora kinase and other kinases involved in mitosis and cytokinesis regulation;
i) proteasome inhibitors (for example lactacystin, bortezomib, epoxomicin);
j) HSP90 inhibitors (for example but not limited to AT-13387, KOS-953, KOS-1022, CNF-1010, CNF-2024, SNX 5422, STA-9090, NVP-HSP990, NVP-AUY922, PU-H17 and XL-888)
k) Selective COX-2 inhibitors (for example celecoxib), or non selective NSAIDs (for example diclofenac, flurbiprofen, ibuprofen, ketoprofen, or naproxen).

In another aspect, a compound of general formula **(I)** can be used in combination with radiation therapy. In yet another aspect, a compound of general formula **(I)** may be administered in combination with standard chemotherapy combinations such as, but not restricted to, CMF (cyclophosphamide, methotrexate and 5-fluorouracil), CAF (cyclophosphamide, doxorubicin and 5-fluorouracil), AC (doxorubicin and cyclophosphamide), FEC (5-fluorouracil, epirubicin, and cyclophosphamide), ACT or ATC (doxorubicin, cyclophosphamide, and paclitaxel), or CMFP (cyclophosphamide, methotrexate, 5-fluorouracil and prednisone).

The invention also provides pharmaceutical compositions comprising one or more compounds of this invention and one or more pharmaceutically acceptable excipient and/or diluent. The pharmaceutical compositions can be chosen on the basis of the treatment requirements. Such compositions are prepared by blending and are suitably adapted to oral or parenteral administration, and as such can be administered in the form of tablets, capsules, oral preparations, powders, granules, pills, injectable, or infusible liquid solutions, suspensions, or suppositories.

Tablets and capsules for oral administration are normally presented in unit dose form and contain conventional excipients such as binders, fillers (including cellulose, mannitol, lactose), diluents, tableting agents, lubricants (including magnesium stearate), detergents, disintegrants (e.g. polyvinylpyrrolidone and starch derivatives such as sodium glycolate starch), coloring agents, flavoring agents, and wetting agents (for example sodium lauryl sulfate).

The oral solid compositions can be prepared by conventional methods of blending, filling or tableting. The blending operation can be repeated to distribute the active principle throughout compositions containing large quantities of fillers. Such operations are conventional.

Oral liquid preparations can be in the form of, for example, aqueous or oily suspensions, solutions, emulsions, syrups or elixirs, or can be presented as a dry product for reconstitution with water or with a suitable vehicle before use. Such liquid preparations can contain conventional additives such as suspending agents, for example sorbitol, syrup, methyl cellulose, gelatin, hydroxyethyl cellulose, carboxymethyl cellulose, aluminium stearate gel, or hydrogenated edible fats; emulsifying agents, such as lecithin, sorbitan monooleate, or acacia; non-aqueous vehicles (which can include edible oils), such as almond oil, fractionated coconut oil, oily esters such as esters of glycerine, propylene glycol, or ethyl alcohol; preservatives, such as methyl or propyl p-hydroxybenzoate or sorbic acid, and if desired, conventional flavoring or coloring agents. Oral formulations also include conventional slow-release formulations such as enterically coated tablets or granules.

Pharmaceutical preparation for administration by inhalation can be delivered from an insufflator or a nebulizer pressurized pack.

For parenteral administration fluid unit dosages can be prepared, containing the compound and a sterile vehicle. The compound can be either suspended or dissolved, depending on the vehicle and concentration. The parenteral solutions are normally prepared by dissolving the compound in a vehicle, sterilising by filtration, filling suitable vials and sealing. Advantageously, adjuvants such as local anaesthetics, preservatives and buffering agents can also be dissolved in the vehicle. To increase the stability, the composition can be frozen after having filled the vials and removed the water under vacuum. Parenteral suspensions are prepared in substantially the same manner, except that the compound can be suspended in the vehicle instead of being dissolved, and sterilized by exposure to ethylene oxide before suspending in the sterile vehicle. Advantageously, a surfactant or wetting agent can be included in the composition to facilitate uniform distribution of the compound of the invention.

For buccal or sublingual administration the compositions may be tablets, lozenges, pastilles, or gel.

The compounds can be pharmaceutically formulated as suppositories or retention enemas, e.g. containing conventional suppositories bases such as cocoa butter, polyethylene glycol, or other glycerides, for a rectal administration.

Another means of administering the compounds of the invention regards topical treatment. Topical formulations can contain for example ointments, creams, lotions, gels, solutions, pastes and/or can contain liposomes, micelles and/or microspheres. Examples of ointments include oleaginous ointments such as vegetable oils, animal fats, semisolid hydrocarbons, emulsifiable ointments such as hydroxystearin sulfate, anhydrous lanolin, hydrophilic petrolatum, cetyl alcohol, glycerol monostearate, stearic acid, water soluble ointments containing polyethylene glycols of various molecular weights. Creams, as known to formulation experts, are viscous liquids or semisolid emulsions, and contain an oil phase, an emulsifier and an aqueous phase. The oil phase generally contains petrolatum and an alcohol such as cetyl or stearic alcohol. Formulations suitable for topical administration to the eye also include eye drops, wherein the active ingredient is dissolved or suspended in a suitable carrier, especially an aqueous solvent for the active ingredient.

A further method of administering the compounds of the invention regards transdermal delivery. Typical transdermal formulations comprise conventional aqueous and non-aqueous vectors, such as creams, oils, lotions or pastes or can be in the form of membranes or medicated patches.

A reference for the formulations is the book by Remington ("Remington: The Science and Practice of Pharmacy", Lippincott Williams & Wilkins, 2000).

The compounds of the present invention may be employed alone as a sole therapy or in combination with other therapeutic agents (see the list of additional agents is as indicated previously and comprises also standard chemotherapeutic agents) for the treatment of the above-mentioned conditions. The combination can be administered as separate compositions (simultaneous, sequential) of the individual components of the treatment or as a single dosage form containing both agents. When the compounds of this invention are in combination with others active ingredients, the active ingredients may be separately formulated into single-ingredient preparations of one of the above-described forms and then provided as combined preparations, which are given at the same time or different times, or may be formulated together into a two- or more-ingredient preparation.

Compounds of general formula **(I)** may be administered to a patient in a total daily dose of, for example, from 0.001 to 1000 mg/kg body weight daily. Dosage unit compositions may contain such amounts of submultiples thereof to make up the daily dose. The determination of optimum dosages for a particular patient is well known to one skilled in the art.

As is common practice, the compositions are normally accompanied by written or printed instructions for use in the treatment in question.

The following examples and biological data are presented in order to further illustrate the invention.

### Detailed description of the Invention

### 1. CHEMICAL SYNTHESIS

Unless otherwise indicated, commercially available reagents and solvents (HPLC grade) were used without further purification. Specifically, the following abbreviations may have been used in the descriptions of the experimental methods:

| | |
|---|---|
| NMR (Nuclear Magnetic Resonance) | ¹H (proton) |
| MHz (Megahertz) | Hz (Hertz) |
| HPLC (High Performance Liquid Chromatography) | LC-MS (Liquid Chromatography Mass Spectrum) |
| s (seconds) | min (minutes) |
| h (hours) | mg (milligrams) |
| g (grams) | µL (microlitres) |
| mL (millilitres) | mmol (millimoles) |
| nm (nanometers) | µM (micromolar) |
| M (molarity) | Rₜ (retention time in minutes) |
| RT (room temperature) | MW (microwave) |
| BOC or boc (*tert*-butyloxycarbonyl) | CH₂Cl₂ (dichloromethane) |
| CH₃CN (acetonitrile) | DMF (dimethylformamide) |
| DMSO (dimethyl sulfoxide) | DMSO-d₆ (deuterated dimethyl sulfoxide) |
| Et₂O (diethyl ether) | EtOAc (ethyl acetate) |
| EtOH (ethanol) | HCl (hydrochloric acid) |
| K₂CO₃ (potassium carbonate) | MeOH (methanol) |
| Na₂CO₃ (sodium carbonate) | Na₂SO₄ (sodium sulphate) |
| NH₃ (ammonia) | TEA (triethylamine) |
| *tert*-BuOH (*tert*-butanol) | |

Except where indicated otherwise, all temperatures are expressed in °C (degrees centigrade) or K (Kelvin).

The ¹H-NMR spectra were acquired with a Varian 500 MHz instrument or, if stated explicitly, at 400 MHz on a Bruker AC 400 spectrometer. The chemical shifts are expressed in parts per million (ppm, δ units). The coupling constants are expressed in Hertz (Hz) and the splitting patterns are described as s (singlet), bs (broad signal), d (doublet), t (triplet), q (quartet), quint (quintet), m (multiplet).

The LC-MS analyses were carried out on a Waters Acquity UPLC or Waters Acquity UPLC H-Class linked to with a SQD Single quadrupole (Waters) using an Acquity UPLC BEH C18 (50 x 2.1 mm, 1.7 µm) or Acquity UPLC HSS T3 (50 x 2.1 mm, 1.8 µm) column. Phase A was composed by either Milli-Q water/CH₃CN 95/5 + 0.07% formic acid or Milli-Q water + 0.07% formic acid; Phase B by CH₃CN + 0.05% formic acid; flow rate: 0.6 mL/min; UV detection (DIODE array) from 210 to 400 nm; ESI+ detection in the 100-2000 m/z range.The yields were calculated assuming that products were 100% pure if not stated otherwise.

### Intermediate 1: 3-(2-Oxooxazolidin-3-yl)benzamide

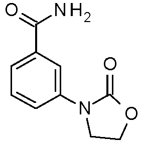

A suspension of 0.56 g (2.7 mmol) 3-(2-oxooxazolidin-3-yl)benzoic acid (WO2003/045913) in 15 mL dry CH₂Cl₂ was treated with 0.246 mL (3.38 mmol) thionyl chloride and 2 drops of dry DMF. After stirring at reflux for 2 h, the mixture was cooled to RT and poured in 4 mL NH₃ (28-30% in water). After 1 h the resulting mixture was filtered off to afford a white solid that was washed with water. The aqueous phases were extracted with CH₂Cl₂, and the combined organic layers were dried over Na₂SO₄ and filtered to give 0.549 g of 3-(2-oxooxazolidin-3-yl)benzamide (98%) as a white solid. ¹H NMR (DMSO-d₆) δ (ppm): 8.01 (bs, 1 H), 7.94-7.88 (m, 1 H), 7.84-7.78 (m, 1 H), 7.61 (d, *J*=7.8 Hz, 1 H), 7.52-7.34 (m, 2 H), 4.49-4.39 (m, 2 H), 4.14-3.98 (m, 2 H). MS (ESI): *m*/*z:* 207 [M+H]⁺.

### Intermediate 2: 4-(2-Oxo-1,3-oxazolidin-3-yl)benzamide

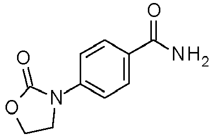

Intermediate 2 was prepared according to the procedure for **Intermediate 1** starting from 4-(2-oxo-1,3-oxazolidin-3-yl)benzoic acid (Enamine, Cat No. EN300-39599). ¹H NMR (DMSO-d₆) δ (ppm): 88.01-7.79 (m, 3 H), 7.67-7.53 (m, 2 H), 7.28 (bs, 1 H), 4.44 (t, *J*=7.6 Hz, 2 H), 4.08 (t, *J*=7.6 Hz, 2 H). MS (ESI): *m*/*z:* 207 [M+H]⁺

The synthesis of 4-(1-methylpiperidin-4-yl)benzamide is described in WO2009055077, of 4-(4-methylpiperazin-1-yl)benzamide in WO2006092510, and of 4-morpholinobenzamide in Eur.J.Med.Chem. 2010, 45, 3709-3718.

### Example 1 N-[4-[trans-2-aminocyclopropyl]phenyl]-4-(4-methylpiperazin-1-yl)benzamide dihydrochloride

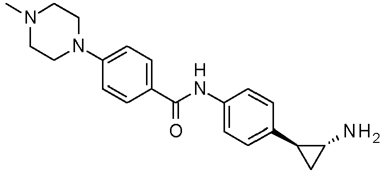

### tert-butyl N-[trans-2-(4-iodophenyl)cyclopropyl]carbamate

7.4 g (27 mmol) Diphenyl phosphoryl azide and 4.4 mL (32 mmol) TEA were added to a solution of 7.05 g (24.5 mmol) of *trans*-2-(4-iodophenyl) cyclopropanecarboxylic acid (J. Med. Chem. 2012, 6624-6628) in 150 mL dry *t*-BuOH. After stirring at 90 °C for 20 h the solution was concentrated and the residue was partitioned between 10% aqueous Na₂CO₃ and Et₂O. The combined organic layers were dried over Na₂SO₄, filtered, and concentrated. The crude product was purified by flash chromatography (hexane/EtOAc 9:1, v;v) giving 5.2 g (57%) of *tert*-butyl *N*-[*trans*-2-(4-iodophenyl)cyclopropyl]carbamate as white solid. ¹H NMR (DMSO-d₆) δ (ppm): 7.67-7.51 (m, 2 H), 7.25 (bs, 1 H), 6.98-6.84 (m, 2 H), 2.57 (bs, 1 H), 1.89-1.74 (m, 1 H), 1.37 (s, 9 H), 1.18-0.95 (m, 2 H). MS (ESI): *m*/*z:* 260 [MH-100]⁺

### N-[trans-2-[4-[[4-(4-methylpiperazin-1-yl)benzoyl]amino]phenyl]cyclopropyl]carbamate

6 mg (0.03 mmol) CuI, 220 mg (0.613 mmol) *tert*-butyl N-[trans-2-(4-iodophenyl)cyclopropyl]carbamate, 0.15 mg (0.67 mmol) 4-(4-methylpiperazin-1-yl)benzamide and 0.17 g (1.23 mmol) K₂CO₃ were placed in a vial and charged with nitrogen. 5 mg (0.06 mmol) *N,N*'-dimethylethane-1,2-diamine and 2 mL dioxane were added with a syringe, and the vial was heated to 110°C for 20 h. The resulting suspension was allowed to cool down to RT and was then filtered through a silica gel pad eluting with 25 ml of 9:1 CH₂Cl₂/MeOH. The filtrate was concentrated and the residue was purified by chromatography (CH₂Cl₂ to CH₂Cl₂/MeOH 95/5) to give 223 mg (81%) of *N*-[*trans*-2-[4-[[4-(4-methylpiperazin-1-yl)benzoyl]amino]phenyl]cyclopropyl]-carbamate as a white solid. ¹H NMR (DMSO-d₆) δ (ppm): 9.86 (s, 1 H), 7.92-7.77 (m, 2 H), 7.69-7.57 (m, 2 H), 7.22 (bs, 1 H), 7.08-6.90 (m, 4 H), 3.31-3.15 (m, 4 H), 2.62-2.53 (m, 1 H), 2.47-2.36 (m, 4 H), 2.22 (s, 3 H), 1.90-1.79 (m, 1 H), 1.38 (s, 9 H), 1.13-1.02 (m, 2 H). MS (ESI): *m*/*z:* 451 [M+H]⁺.

### N-[4-[trans-2-aminocyclopropyl]phenyl]-4-(4-methylpiperazin-1-yl)benzamide dihydrochloride

2 mL of 2 M HCl in Et₂O was added to a solution of 200 mg (0.44 mmol) of *N-[trans-2-*[4-[[4-(4-methylpiperazin-1-yl)benzoyl]amino]phenyl]cyclopropyl]carbamate in 3 mL Et₂O/MeOH (2:1, v:v) cooled down to 0°C. After stirring at RT for 20 h the formed precipitate was filtered off and the light yellow solid washed with Et₂O and dried at 40°C under vacuum giving 167 mg (89%) of *N*-[4-[*trans*-2-aminocyclopropyl]phenyl]-4-(4-methylpiperazin-1-yl)benzamide as its dihydrochloride salt. ¹H NMR (DMSO-d₆) δ (ppm): 9.97 (s, 1 H), 9.75 (bs, 1 H), 8.22 (bs, 3 H), 7.98-7.83 (m, 2 H), 7.77-7.63 (m, 2 H), 7.20-6.98 (m, 4 H), 4.14-3.93 (m, 2 H), 3.60-3.45 (m, 2 H), 3.18-2.98 (m, 4 H), 2.87 (s, 3 H), 2.83-2.74 (m, 1 H), 2.27-2.17 (m, 1 H), 1.35-1.27 (m, 1 H), 1.24-1.14 (m, 1 H). MS (ESI): *m*/*z:* 351 [M+H]⁺.

According to the procedure described for Example **1,** Examples **2, 3, 4** and **5** were synthesized starting from the appropriate benzamide and *tert*-butyl *N*-[trans-2-(4-iodophenyl)cyclopropyl]carbamate.

### Example 2 N-[4-[trans-2-aminocyclopropyl]phenyl]-4-(1-methyl-4-piperidyl)benzamide hydrochloride.

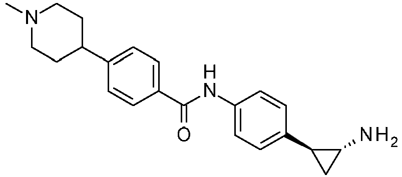

¹H NMR (DMSO-d₆) δ (ppm): 10.17 (s, 1 H), 9.39 (bs, 1 H), 8.23 (bs, 3 H), 7.98-7.87 (m, 2 H), 7.73-7.61 (m, 2 H), 7.48-7.31 (m, 2 H), 7.19-7.09 (m, 2 H), 3.61-3.45 (m, 2 H), 3.14-2.99 (m, 2 H), 2.94-2.75 (m, 5 H), 2.30-2.19 (m, 1 H), 2.12-1.98 (m, 2 H), 1.93-1.78 (m, 2 H), 1.36-1.28 (m, 1 H), 1.23-1.14 (m, 1 H). MS (ESI): m/z: 316 [M+H]⁺.

### Example 3 N-[4-[trans-2-aminocyclopropyl]phenyl]-3-(2-oxooxazolidin-3-yl)benzamide hydrochloride.

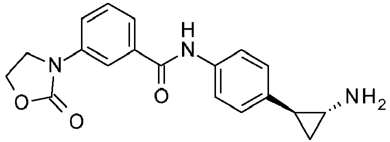

¹H NMR (DMSO-d₆) δ (ppm): 10.29 (s, 1 H), 8.31 (bs, 3 H), 8.07-8.00 (m, 1 H), 7.84-7.77 (m, 1 H), 7.73-7.66 (m, 3 H), 7.59-7.50 (m, 1 H), 7.19-7.12 (m, 2 H), 4.51-4.43 (m, 2 H), 4.20-4.08 (m, 2 H), 2.87-2.75 (m, 1 H), 2.32-2.21 (m, 1 H), 1.39-1.29 (m, 1 H), 1.27-1.15 (m, 1 H). MS (ESI): m/z: 210 [M+H]⁺.

### Example 4 N-[4-[trans-2-aminocyclopropyl]phenyl]-4-morpholino-benzamide hydrochloride.

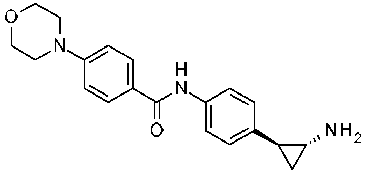

¹H NMR (DMSO-d₆) δ (ppm): 9.93 (s, 1 H), 8.31 (bs, 3 H), 7.93-7.81 (m, 2 H), 7.74-7.62 (m, 2 H), 7.17-7.07 (m, 2 H), 7.05-6.96 (m, 2 H), 3.79-3.68 (m, 4 H), 3.29-3.19 (m, 4 H), 2.85-2.71 (m, 1 H), 2.31-2.19 (m, 1 H), 1.40-1.27 (m, 1 H), 1.23-1.14 (m, 1 H). MS (ESI): m/z: 292 [M+H]⁺.

### Example 5 N-[4-[trans-2-aminocyclopropyl]phenyl]-4-(2-oxooxazolidin-3-yl)benzamide hydrochloride.

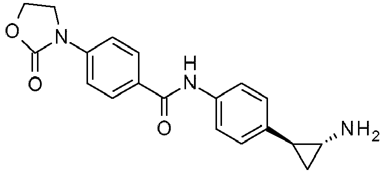

¹H NMR (DMSO-d₆) δ (ppm): 10.16 (s, 1 H), 8.12 (bs, 3 H), 8.02-7.96 (m, 2 H), 7.74-7.66 (m, 4 H), 7.18-7.08 (m, 2 H), 4.55-4.40 (m, 2 H), 4.20-4.02 (m, 2 H), 2.85-2.74 (m, 1 H), 2.31-2.15 (m, 1 H), 1.36-1.13 (m, 2 H). MS (ESI): m/z: 308 [M+H]⁺.

### Example 6 Benzyl N-[4-[[4-[trans-2-aminocyclopropyl]phenyl]carbamoyl]-2-(4-methylpiperazin-1-yl)phenyl]carbamate dihydrochloride

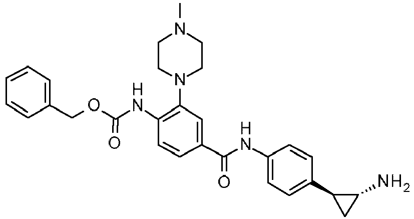

### tert-Butyl 3-(4-methylpiperazin-1-yl)-4-nitrobenzoate

A suspension of *tert* butyl 3-chloro-4-nitro-benzoate (7.76 mmol, 2 g, (Bujok, R. et al.. Tetrahedron 2010, 66, 698-708), dry K₂CO₃ (23.3 mmol, 3.22 g) and *N-*methylpiperazine (23.3 mmol, 2.58 mL) was stirred in dry DMF (10 mL) at 90°C for 5 h in a sealed tube. After this time the reaction mixture was quenched with water (50 mL) and extracted with EtOAc, washed with brine and dried over Na₂SO₄. The collected organic phases were concentrated and the residue was purified on silica gel (eluent: EtOAc) to obtain *tert*-butyl 3-(4-methylpiperazin-1-yl)-4-nitrobenzoate as a yellow solid. ¹H NMR (CDCl₃, 400 MHz) δ (ppm): 1.60 (s, 9H, -COOC(C*H*₃)₃), 2.37 (s, 3H, -NC*H*₃), 2.59 (t, 4H, CH₃N(C*H*₂)₂), 3.20 (t, 4H, -PhN(C*H*₂)₂), 7.07-7.09 (d, 1 H, benzene proton), 8.03-8.05 (d, 1 H, benzene proton), 8.37 (s, 1 H, benzene proton); ¹³C NMR (CDCl₃, 100 MHz) δ (ppm): 28.8 (3C), 46.8, 51.5 (2C), 57.4 (2C), 81.3, 113.7, 120.6, 124.7, 137.3, 139.8, 143.5, 164.7; MS (EI) *m*/*z*: 321.17 [M]⁺.

### tert-Butyl 4-amino-3-(4-methylpiperazin-1-yl)benzoate

A suspension of *tert*-butyl 3-(4-methylpiperazin-1-yl)-4-nitrobenzoate (2.5 mmol, 0.8 g) in MeOH (30 mL) and 10% palladium on carbon (0.12 mmol, 0.13 g) were placed in a Parr apparatus and was hydrogenated at 50 psi and 25°C for 5 h. The palladium was then filtered off and the MeOH was evaporated to afford an oily residue that was purified on silica gel (eluent: CHCl₃/MeOH, 10:1, v:v) to provide *tert*-butyl 4-amino-3-(4-methylpiperazin-1-yl)benzoate as a yellow solid. ¹H NMR (CDCl₃, 400 MHz) δ (ppm): 1.58 (s, 9H, -COOC(C*H*₃)₃), 2.39 (s, 3H, -NC*H*₃), 2.60 (t, 4H, -N(C*H*₂)₂), 2.97 (t, 4H, - PhN(C*H*₂)₂), 4.35 (bs, 2H, -PhN*H*₂), 6.68-6.70 (d, 1 H, benzene proton), 7.62-7.64 (d, 1 H, benzene proton), 7.71 (s, 1 H, benzene proton); ¹³C NMR (CDCl₃, 100 MHz) δ (ppm): 28.9 (3C), 46.7, 52.4 (2C), 57.2 (2C), 81.9, 113.3, 117.2, 120.5, 121.6, 137.7, 139.1, 164.8; MS (EI) *m*/*z:* 291.19 [M]⁺.

### tert-Butyl 4-(benzyloxycarbonylamino)-3-(4-methylpiperazin-1-yl)benzoate

Benzyl chloroformate (2.1 mmol, 0.3 mL) was slowly added at 0°C to a solution of *tert-*butyl 4-amino-3-(4-methylpiperazin-1-yl)benzoate (1.72 mmol; 0.5 g) in THF (10 mL) and TEA (2.1 mmol, 0.29 mL). The resulting mixture was stirred at RT for 1.5 h, then it was quenched with water (20 mL) and extracted with CH₂Cl₂ (3 x 20 mL). The organic phases were washed with brine, dried over Na₂SO₄ and concentrated to afford a residue that was purified on silica gel (eluent EtOAc/CHCl₃, 1:1, v:v) providing *tert*-butyl 4-(benzyloxycarbonylamino)-3-(4-methylpiperazin-1-yl)benzoate (72%). ¹H NMR (CDCl₃, 400 MHz) δ (ppm): 1.60 (s, 9H, -COOC(C*H*₃)₃), 2.39 (s, 3H, -NC*H*₃), 2.62 (t, 4H, CH₃N(C*H*₂)₂), 2.92 (t, 4H, -PhN(C*H*₂)₂), 5.28 (s, 2H, -NHCOOC*H*₂Ph), 7.15-7.17 (d, 1 H, benzene proton), 7.37-7.47 (m, 5H, -CH₂*Ph*), 7.69-7.72 (m, 2H, benzene protons), 8.69 (bs, 1 H, -NHCOOCH₂Ph); ¹³C NMR (CDCl₃, 100 MHz) δ (ppm): 28.9 (3C), 46.3, 52.4 (2C), 57.5 (2C), 66.7, 81.4, 112.4, 119.9, 122.6, 125.3, 126.7, 127.3 (2C), 127.4, 129.1 (2C), 136.5, 142.7, 153.6, 164.7; MS (EI) *m*/*z:* 425.23 [M]⁺.

### 4-(benzyloxycarbonylamino)-3-(4-methylpiperazin-1-yl)benzoic acid

A solution of *tert*-butyl 4-(benzyloxycarbonylamino)-3-(4-methylpiperazin-1-yl)benzoate (0.47 mmol, 0.2 g) and TFA (9.4 mmol, 0.72 mL) in dry CH₂Cl₂ (5 mL) was stirred at RT overnight. The solvent was removed and the resulting solid was triturated with Et₂O (10 mL) to give 4-(benzyloxycarbonylamino)-3-(4-methylpiperazin-1-yl)benzoic acid as a colorless solid (83%). ¹H NMR (DMSO-*d*₆, 400 MHz) δ (ppm): 2.87 (s, 3H, -NC*H*₃), 2.99-3.12 (m, 4H, CH₃N(C*H*₂)₂), 3.46-3.48 (t, 4H, -PhN(C*H*₂)₂), 5.23 (s, 2H, -NHCOOC*H*₂Ph), 7.36-7.47 (m, 5H, -CH₂*Ph*), 7.70 (s, 1 H, benzene proton), 7.75-7.77 (d, 1 H, benzene proton), 8.04-8.06 (d, 1 H, benzene proton), 8.76 (bs, 1 H, -N*H*COOCH₂Ph), 9.72 (bs, 1 H, N*H*⁺), 12.88 (bs, 1 H, -COO*H*); ¹³C NMR (DMSO-*d*₆, 400 MHz) δ (ppm): 46.7, 52.4 (2C), 57.2 (2C), 66.9, 112.5, 120.4, 122.7, 124.6, 126.2, 127.3 (2C), 127.5, 128.8 (2C), 136.3, 142.7, 154.1, 166.8; MS (EI) *m*/*z*: 369.17 [M]⁺.

According to the procedure described for the preparation of 4-(benzyloxycarbonylamino)-3-(4-methylpiperazin-1-yl)benzoic acid the following intermediate compounds **(Table 1)** were synthesized starting from *tert* butyl 4-chloro-3-nitro-benzoate.

**Table 1:**

| Molecular structure | Recrystal. Solvent, where necessary | m.p. (°C) | Yield (%) |
|---|---|---|---|
| 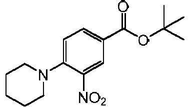 | toluene | 144-146 | 88 |
| 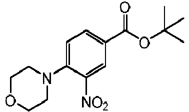 | toluene | 149-151 | 85 |
| 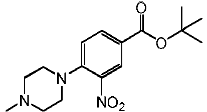 | toluene | 138-140 | 92 |
| 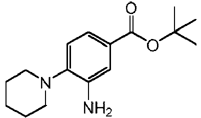 | cyclohexane | 115-117 | 67 |
| 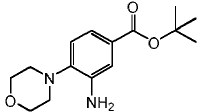 | cyclohexane | 126-128 | 73 |
| 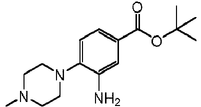 | cyclohexane | 114-116 | 65 |
| 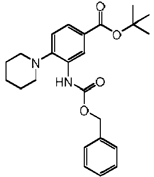 | - | oil | 68 |
| 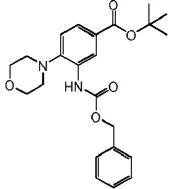 | - | oil | 71 |
| 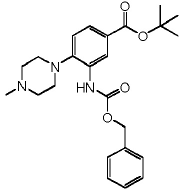 | - | oil | 65 |
| 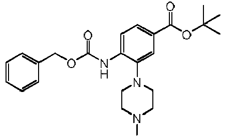 | - | oil | 72 |
| 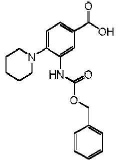 | acetonitrile | 214-216 | 86 |
| 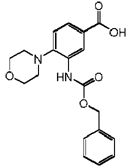 | acetonitrile | 234-236 | 83 |
| 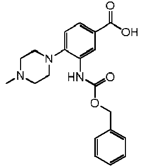 | acetonitrile | 205-207 | 76 |

### Benzyl N-[4-[[4-[trans-2-(tert-butoxycarbonylamino)cyclopropyl]phenyl]carbamoyl]-2-(4-methylpiperazin-1-yl)phenyl]carbamate

TEA (1.08 mmol, 0.15 mL) and PyBop (0.32 mmol, 0.169 g) were added to a solution of 4-(benzyloxycarbonylamino)-3-(4-methylpiperazin-1-yl)benzoic acid (0.27 mmol, 0.1 g) in dry DMF under nitrogen atmosphere, and the resulting mixture was stirred at RT for 45 min. *tert*-Butyl *N*-[*trans*-2-(4-aminophenyl)cyclopropyl]carbamate (2.71 mmol, 0.067 g) was added under N₂ atmosphere and the reaction was continued overnight, then quenched with water (20 mL) and the product was extracted with CH₂Cl₂ (3 x 20 mL). The organic phases were washed with brine, dried over Na₂SO₄ and concentrated to furnish a residue that was purified by chromatography on silica gel (eluent: EtOAc/CHCl₃, 1:1, v:v) giving benzyl *N*-[4-[[4-[*trans*-2-(tert-butoxycarbonylamino)cyclopropyl]phenyl]carbamoyl]-2-(4-methylpiperazin-1-yl)phenyl]carbamate (74%). ¹H NMR (CDCl₃, 400 MHz) δ (ppm): 1.18 (m, 1H, CH*H* cyclopropane), 1.37 (m, 1 H, C*H*H cyclopropane), 1.5 (s, 9H, -C(C*H*₃)₃), 2.31 (m, 1 H, C*H*NH₂), 2.80 (m, 4H, CH₃N(C*H*₂)₂), 3.15 (m, 4H, -PhN(C*H*₂)₂), 3.32-3.38 (m, 4H, -NC*H*₃ and PhC*H*), 4.89 (bs, 1 H, N*H*COOC(CH₃)₃), 5.23 (s, 2H, -NHCOOC*H*₂Ph), 7.13-7.16 (d, 2H, benzene protons), 7.34-7.48 (m, 5H, benzene protons), 7.70-7.72 (d, 2H, benzene protons), 7.79-7.86 (m, 2H, benzene protons), 8.01-8.03 (d, 1 H, benzene proton), 8.74 (bs, 1H, -N*H*COOCH₂Ph), 10.27 (bs, 1H, PhCON*H*), 11.12 (bs, 1H, N*H*⁺); ¹³C NMR (CDCl₃, 100 MHz) δ (ppm): 14.5, 23.1, 28.6 (3C), 32.7, 46.8, 52.3 (2C), 57.4 (2C), 66.5, 79.8, 110.3, 119.5, 121.6 (2C), 122.6, 124.5, 125.3 (2C), 127.6 (2C), 127.9, 129.3 (2C), 130.8, 134.5, 136.4, 137.5, 143.3, 153.7, 155.8, 164.9; MS (EI) *m*/*z:* 599.31 [M]⁺.

According to the procedure described for the preparation benzyl *N*-[4-[[4-[*trans*-2-(tert-butoxycarbonylamino)cyclopropyl]phenyl]carbamoyl]-2-(4-methylpiperazin-1-yl)phenyl]carbamate the following intermediate compounds were synthesized starting from the corresponding carboxylic acid and *tert*-butyl *N*-[*trans*-2-(4-aminophenyl)cyclopropyl]carbamate **(Table 2).**

**Table 2:**

| Molecular structure | Recrystal. solvent | m.p. (°C) | Yield (%) |
|---|---|---|---|
| 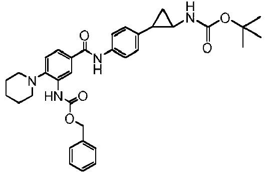 | cyclohexane | 116-118 | 72 |
| 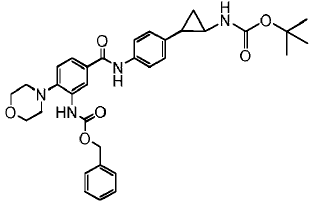 | cyclohexane | 118-120 | 75 |
| 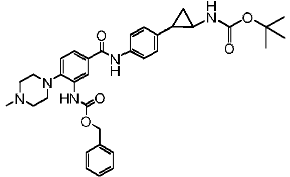 | cyclohexane | 125-127 | 68 |

### Benzyl N-[4-[[4-[trans-2-aminocyclopropyl]phenyl]carbamoyl]-2-(4-methylipiperazin-1-yl)phenyl]carbamate dihydrochloride

A solution of benzyl *N*-[4-[[4-[*trans*-2-(tert-butoxycarbonylamino)cyclopropyl]-phenyl]carbamoyl]-2-(4-methylpiperazin-1-yl)phenyl]carbamate (0.1 mmol, 0.06 g) and 4 M HCl (2.0 mmol, 0.5 mL) was stirred at RT overnight. The solid was then filtered off and washed with Et₂O (3 x 5 mL) to afford benzyl *N*-[4-[[4-[*trans*-2-aminocyclopropyl]phenyl]carbamoyl]-2-(4-methylpiperazin-1-yl)phenyl]carbamate dihydrochloride as colorless solid (79%). ¹H NMR (DMSO-*d*₆, 400 MHz) δ (ppm): 1.20 (m, 1 H, CH*H* cyclopropane), 1.39 (m, 1 H, C*H*H cyclopropane), 2.34 (m, 1H, C*H*NH₂), 2.80 (m, 4H, CH₃N(C*H*₂)₂), 3.15 (m, 4H, -PhN(C*H*₂)₂), 3.32-3.38 (m, 4H, -NC*H*₃ and PhC*H*), 5.23 (s, 2H, -NHCOOC*H*₂Ph), 7.13-7.16 (d, 2H, benzene protons), 7.34-7.48 (m, 5H, benzene protons), 7.70-7.72 (d, 2H, benzene protons), 7.79-7.86 (m, 2H, benzene protons), 8.01-8.03 (d, 1 H, benzene proton), 8.56 (bs, 3H, N*H*₃⁺), 8.74 (bs, 1 H, -NHCOOCH₂Ph), 10.27 (bs, 1 H, PhCON*H*), 11.12 (bs, 1 H, N*H*⁺) ppm; ¹³C NMR (DMSO-*d*₆, 400 MHz) δ (ppm): 12.3, 20.6, 40.5, 46.8, 52.3 (2C), 57.3 (2C), 66.5, 110.3, 119.7, 121.1 (2C), 122.9, 124.3, 125.5 (2C), 127.2 (2C), 127.5, 129.3 (2C), 130.8, 134.7, 136.3, 137.2, 143.2, 153.8, 164.9 ppm; MS (EI) *m*/*z:* 499.26 [M]⁺.

According to the procedure described for Example **6** the compounds of Examples **7, 8** and **9** were synthesized starting from the appropriate *tert*-butyl *N*-[*trans*-2-(4-aminophenyl)cyclopropyl]carbamate.

### Example 7 Benzyl N-[5-[[4-[(trans-2-aminocyclopropyl]phenyl]carbamoyl]-2-(4-methylpiperazin-1-yl)phenyl]carbamate dihydrochloride.

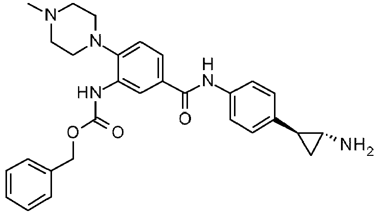

¹H NMR (DMSO-d₆, 400 MHz) δ (ppm): 1.20 (m, 1 H, CHH cyclopropane), 1.39 (m, 1 H, CHH cyclopropane), 2.34 (m, 1 H, CHNH₂), 2.83 (t, 4H, CH₃N(CH₂)₂), 3.15 (m, 4H, PhN(CH₂)₂), 3.33 (s, 3H, -NCH₃), 5.23 (s, 2H, -NHCOOCH₂Ph), 7.15-7.18 (d, 2H, benzene protons), 7.30-7.48 (m, 5H, benzene protons), 7.70-7.72 (d, 2H, benzene protons), 7.79-7.86 (m, 2H, benzene protons), 8.01-8.03 (d, 1 H, benzene proton), 8.56 (bs, 3H, N*H*₃⁺), 8.74 (bs, 1 H, -NHCOOCH₂Ph), 10.27 (bs, 1 H, PhCONH), 11.12 (bs, 1 H, NH⁺); ¹³C NMR (DMSO-d₆, 100 MHz) δ (ppm): 17.3, 25.6, 34.4, 46.7, 52.1 (2C), 57.3 (2C), 66.8, 114.7, 118.2, 121.0, 121.3 (2C), 123.9, 125.5 (2C), 125.9, 127.4 (2C), 127.9, 129.0 (2C), 134.5, 136.3, 137.3, 146.4, 153.8, 164.8. MS (ESI) *m*/*z:* 500 ([M+H]⁺).

### Example 8 Benzyl N-[5-[[4-[trans-2-aminocyclopropyl]phenyl]carbamoyl]-2-(1-piperidyl)phenyl]carbamate hydrochloride

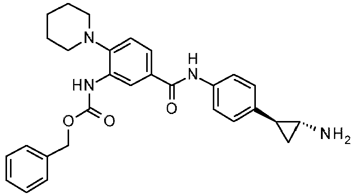

¹H NMR (DMSO-d₆, 400 MHz) δ (ppm): 1.20 (m, 1 H, CHH cyclopropane), 1.39 (m, 1 H, CHH cyclopropane), 1.52-1.59 (m, 6H, piperidine protons), 2.34 (m, 1 H, CHNH2), 3.0 (t, 4H, -N(CH₂)₂), 3.32-3.36 (m, 1 H, PhCH), 5.23 (s, 2H, -NHCOOCH₂Ph), 7.14-7.17 (d, 2H, benzene protons), 7.32-7.46 (m, 5H, benzene protons), 7.68-7.70 (d, 2H, benzene protons), 7.79-7.86 (m, 2H, benzene protons), 8.02-8.04 (d, 1 H, benzene proton), 8.56 (bs, 3H, NH₃⁺), 8.74 (bs, 1H, -NHCOOCH2Ph), 10.30 (bs, 1H, PhCONH), 11.12 (bs, 1H, NH⁺); ¹³C NMR (DMSO-d₆, 100 MHz) δ (ppm): 17.2, 24.5, 25.5 (2C), 25.7, 34.4, 54.7 (2C), 66.8, 114.8, 118.5, 120.8, 121.3 (2C), 124.0, 125.4 (2C), 125.9, 127.5 (2C), 127.7, 128.9 (2C), 134.5, 136.2, 137.3, 146.5, 153.9, 164.8. MS (ESI) *m*/*z:* 485 ([M+H]⁺).

### Example 9 Benzyl N-[5-[[4-[trans-2-aminocyclopropyl]phenyl]carbamoyl]-2-morpholino-phenyl]carbamate hydrochloride.

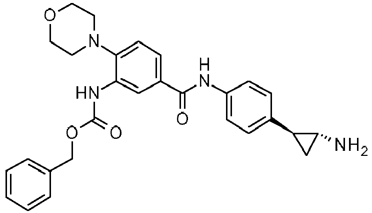

¹H NMR (DMSO-d₆, 400 MHz) δ (ppm): 1.21 (m, 1 H, CHH cyclopropane), 1.38 (m, 1 H, CHH cyclopropane), 2.33 (m, 1 H, CHNH2), 2.89 (t, 4H, -N(CH₂)₂), 3.88 (m, 4H, O(CH₂)₂), 5.27 (s, 2H, -NHCOOCH₂Ph), 7.15-7.18 (d, 2H, benzene protons), 7.32-7.46 (m, 5H, benzene protons), 7.71-7.73 (d, 2H, benzene protons), 7.79-7.86 (m, 2H, benzene protons), 8.03-8.05 (d, 1 H, benzene proton), 8.58 (bs, 3H, NH₃⁺), 8.75 (bs, 1 H, -NHCOOCH₂Ph), 10.30 (bs, 1 H, PhCONH), 11.13 (bs, 1 H, NH⁺). MS (ESI) *m*/*z:* 487 ([M+H]⁺).

### 2. BIOLOGICAL TESTING

### 2.1 Assay of enzyme inhibition of KDM1A (LSD1)

The complex of human recombinant KDM1A (LSD1)/CoRest protein was produced in E. coli as separate proteins and co-purified following previously reported procedures (Forneris F. et al. Trends Biochem. Sci. 2008, 33, 181-189; Forneris F. et al. J.Biol.Chem. 2007, 282, 20070-20074). The experiments were performed using a mono-methylated H3-K4 peptide containing 21 amino acids (custom synthesis done by Thermo Scientific) as substrate and in 50 mM TRIS, pH 8.0 and 0.05 mg/ml BSA. The peptide purity was >90% as checked by analytical high-pressure liquid chromatography and mass spectrometry.

The demethylase activity was estimated under aerobic conditions and at RT by measuring the release of H₂O₂ produced during the catalytic process by the Amplex® UltraRed detection system coupled with peroxidase assay. Briefly, a fixed amount of KDM1A/CoRest complex was incubated at RT for 15 min in the absence and/or the presence of various concentrations of inhibitor (e.g. from 0 to 100 µM, depending on the inhibitor strength) and of Amplex® UltraRed detection system coupled with peroxidase assay. The inhibitors were tested twice in duplicates at each concentration. Tranylcypromine (Sigma) was used as control. After preincubation of the enzyme with the inhibitor, 4.5 µM of mono-methylated H3-K4 peptide was added and the experiment was left for additional 12 min. The conversion of the Amplex® Ultra Red reagent to resorufin was monitored in continuous by fluorescence (excitation at 540 nm, emission at 590 nm) using a microplate reader (Infinite 200, Tecan). Arbitrary units were used to measure the level of H₂O₂ produced in the absence and/or in the presence of inhibition. The maximum demethylase activity of KDM1A/CoRest was obtained in the absence of inhibitors and corrected for background fluorescence in the absence of KDM1A/CoRest. The IC₅₀ was calculated using GraphPad Software.

Compounds 1-9 exhibited IC₅₀ values of less than 1.0 µM.

### 2.2 Cell growth

CellTiter-Flor^{®} (Promega) is as a nonlytic, single-reagent-addition fluorescence assay that measures the relative number of living cells in a culture population after experimental manipulation. The CellTiter-Fluor™ Cell Viability Assay measures the conserved and constitutive protease activity within live cells and therefore acts as a marker for cell viability.

Human leukemia MV4-11 cells, (obtained from the Deutsche Sammlung von Mikroorganismen und Zellkulturen, **ACC 102)** in exponential growth, were incubated for 48 h with different concentrations of the inhibitors. After 48 h a volume of CellTiter-Fluor^{®} Reagent equal to one fifth of volume of cell culture medium was added. The content was mixed and incubates for at least 90 min at 37°C degree to obtain a stable signal. The fluorescence was recorded using an excitation wavelength of 360 nm and an emission at 535 nm. The IC₅₀ was calculated using GraphPad Software.

The obtained results are illustrated in **Table 3.** IC₅₀ results were allocated to one of 3 ranges as follows: Range A: IC₅₀ from 50 to 100 µM; Range B: from 5 to 50 µM; Range C: IC₅₀ ≤ 5 µM.

**Table 3: Results of the cell growth inhibitory assay:**

| Cpd | Name | Structure | IC₅₀ [µM] |
|---|---|---|---|
| **5b*** | *N*-[4-[*trans*-2-aminocyclopropyl]phenyl]benzamide | 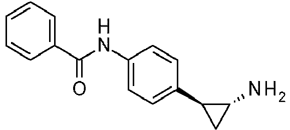 | A |
| 1 | *N-*[4-[*trans*-2-aminocyclopropyl]phenyl]-4-(4-methylpiperazin-1-yl)benzamide dihydrochloride | 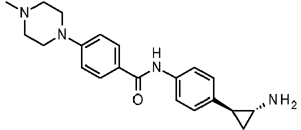 | C |
| 2 | *N-*[4-[*trans*-2-aminocyclopropyl]phenyl]-4-(1-methyl-4-piperidyl)benzamide hydrochloride | 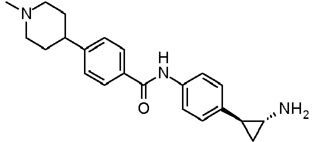 | C |
| 3 | *N*-[4-[*trans*-2-aminocyclopropyl]phenyl]-3-(2-oxooxazolidin-3-yl)benzamide hydrochloride | 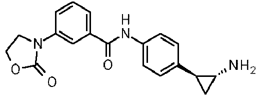 | C |
| 4 | *N*-[4-[*trans-*2-aminocyclopropyl]phenyl]-4-morpholino-benzamide hydrochloride | 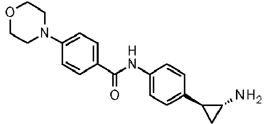 | B |
| 5 | *N*-[4-[*trans*-2-aminocyclopropyl]phenyl]-4-(2-oxooxazolidin-3-yl)benzamide hydrochloride | 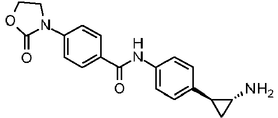 | B |
| 6 | benzyl *N*-[4-[[4-[*trans*-2-aminocyclopropyl]phenyl]carbamoyl]-2-(4-methylpiperazin-1-yl)phenyl]carbamate dihydrochloride | 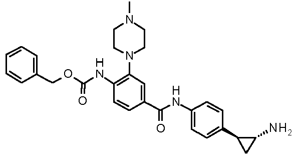 | C |
| 7 | benzyl *N*-[5-[[4-[(*trans*-2-aminocyclopropyl]phenyl]carbamoyl]-2-(4-methylpiperazin-1-yl)phenyl]carbamate dihydrochloride | 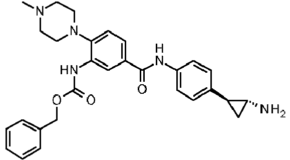 | C |
| 8 | benzyl *N-*[5-[[4-[*trans*-2-aminocyclopropyl]phenyl]carbamoyl]-2-(1-piperidyl)phenyl]carbamate hydrochloride | 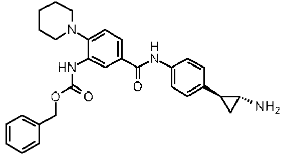 | C |
| 9 | benzyl *N-*[5-[[4-[*trans*-2-aminocyclopropyl]phenyl]carbamoyl]-2-morpholinophenyl]carbamate hydrochloride | 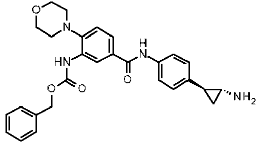 | C |

| | | | |
|---|---|---|---|
| * compound **5b** of WO2011/131576 | | | |

### 2.3 Bioluminescent-Coupled Assay for Monoamine oxidases (MAO-Glo Assay)

The MAO-Glo Assay from Promega (cat. V1402, Promega, Madison, WI) was used to measure the effect of inhibitors on MAO-A and MAO-B activity.

Human recombinant MAO A and MAO B were expressed in *Pichia pastoris* and purified as published (Binda C. et al. Proc. Natl. Acad. Sci. USA, 2003, 9750-9755). The assay was performed at RT in 50 µl (25 µl reaction solution + 25 µl detection reagent) in 96 well half area white plates (cat. 3693, Corning, Corning, NY). Luminescence was measured after 20 min incubation in the dark using a microplate reader (Infinite F200, Tecan Group, Switzerland) with an integration time of 0.25 s per well.

50 nM MAO-A or 125 nM MAO-B were incubated with five different inhibitor concentrations (from 0.004 µM to 100µM) for 15 min at RT in Promega MAO Buffer or Promega MAO-B Buffer (MAO-Glo Assay kit, catalogue number V1402, Promega, Madison, WI). The Promega MAO substrate was at a concentration equal to the calculated Kₘ (40 µM for MAO-A and 14 µM for MAO-B). After 30 min of incubation the reaction was stopped with the Promega detection reagent. The experiments were carried out in duplicate. IC₅₀ was calculated using GraphPad Prism version 4.0 (GraphPad Software, San Diego, CA). In order to determine if any of the compounds inhibit the Luciferin Detection Reagent, each compound was re-screened in the absence of MAOs using 0.5 µM D-luciferin methyl ester as substrate (Michael P. et al. Cell Notes, 2006, 14, 4-7, Promega Corporation and Promega Biosciences, Inc). **Table 4** reports the ratio of the IC₅₀ values against MAO-A over those obtained for LSD1 of compounds of this invention and the representative compound **5b** of PCT application WO2011/131576.

**Table 4**

| Cpd | Name | Structure | IC₅₀ (MAO-A)/ IC₅₀ (LSD1) |
|---|---|---|---|
| **5b*** | *N-*[4-[*trans-*2*-*aminocyclo propyl]phenyl]benzamide | 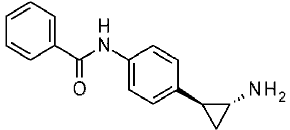 | 1.56 |
| 1 | *N-*[4-[*trans-*2-aminocyclopropyl]phenyl] -4-(4-methylpiperazin-1-yl)benzamide dihydrochloride | 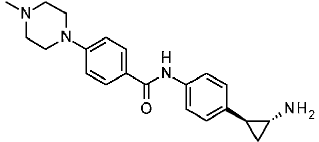 | 3.48 |
| 2 | *N*-[4-[*trans*-2-aminocyclopropyl]phenyl] -4-(1-methyl-4-piperidyl)benzamide hydrochloride | 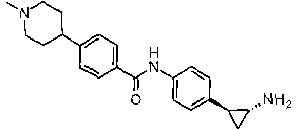 | 5.26 |
| 3 | *N*-[4-[*trans*-2-aminocyclopropyl]phenyl] -3-(2-oxooxazolidin-3-yl)benzamide hydrochloride | 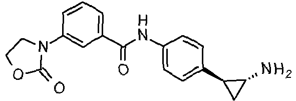 | 3.30 |
| 6 | benzyl *N*-[4-[[4-[*trans*-2-aminocyclopropyl]phenyl] carbamoyl]-2-(4-methylpiperazin-1-yl)phenyl]carbamate dihydrochloride | 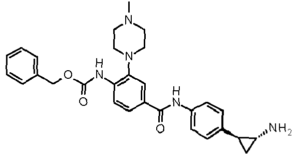 | 15.3 |
| 7 | benzyl *N*-[5-[[4-[(*trans-*2-aminocyclopropyl]phenyl] carbamoyl]-2-(4-methylpiperazin-1-yl)phenyl]carbamate dihydrochloride | 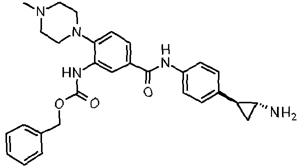 | 11.2 |
| 8 | benzyl *N*-[5-[[4-[*trans-*2-aminocyclopropyl]phenyl] carbamoyl]-2-(1-piperidyl)phenyl]carbamate hydrochloride | 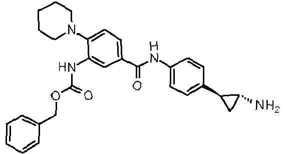 | 2.23 |
| 9 | benzyl *N*-[5-[[4-[*trans*-2-aminocyclopropyl]phenyl] carbamoyl]-2-morpholino-phenyl]carbamate hydrochloride | 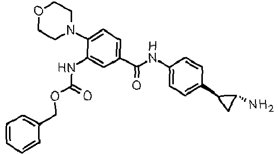 | 3.17 |

| | | | |
|---|---|---|---|
| * compound **5b** of WO2011/131576 | | | |

Further, the compounds of the invention display selectivity over MAO-B.

### 2.4 In vivo activity

The *in vivo* activity was conducted on a mouse model as characterized by Minucci *et al*..(Minucci S. et al. Blood 2002, 100, 2989-2995) The model is characterized by the development of leukemia, resembling the human acute promyelocytic leukemia, which is associated to a blast infiltration of several organs as bone marrow, liver and particularly of the spleen. In the conducted experiment, splenomegaly was studied as read out of blast infiltration and development of leukemia.

For the *in vivo* analysis, one million of leukemic cells (obtained from 129SvEv mice, Minucci S. et al. Blood 2002, 100, 2989-2995, obtained from Taconic, One Hudson City Centre Hudson, NY (USA)) were injected intravenously into non-irradiated syngenic recipients. Treatment started once blast cells are detected in the recipients' peripheral blood (9 days after injection). The compounds were intravenously or orally administered at different doses for at least two weeks. The *in vivo* efficacy of compounds was assessed by determining the increase of mice survival duration between vehicle and compound/s treated mice.

## Claims

1. A compound of formula (**I**) 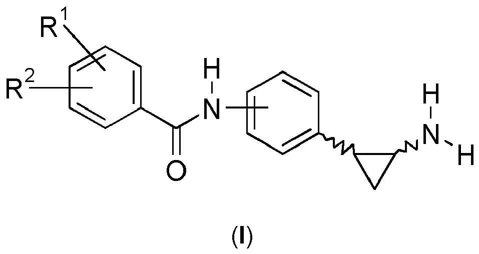 wherein:
**R¹** is heterocyclyl or heterocyclyl substituted by oxo, wherein the heterocyclyl is
unsubstituted or substituted by one or more C₁-C₆ alkyl;
**R²** is hydrogen; halogen; C₁-C₆ alkyl; C₁-C₆ alkoxy; C₁-C₆ haloalkyl; or C₁-C₆
haloalkoxy; or benzyloxycarbonylamino;
or stereoisomers or pharmaceutically acceptable salts thereof.

2. The compound according to claim 1, wherein **R¹** is pyrrolidinyl, piperidinyl, piperazinyl, tetrahydrofuranyl, tetrahydropyranyl, morpholinyl, thiomorpholinyl, tetrahydroquinolinyl, tetrahydroisoquinolinyl, tetrahydroquinoxalinyl, benzodioxolyl, 2,3-dihydro-benzodioxinyl, benzoxazolyl, azepinyl, diazapinyl or 2-oxooxazolidinyl.

3. The compound according to claim 1 or 2, wherein **R¹** is 4-methylpiperazin-1-yl, 1-methylpiperidin-4-yl, piperidin-1-yl, or 2-oxooxazolidin-3-yl.

4. The compound according to any one of the previous claims, wherein **R²** is selected from the group consisting of hydrogen or benzyloxycarbonylamino.

5. The compound according to claim 1 selected from:
*N*-[4-[*trans*-2-aminocyclopropyl]phenyl]-4-(4-methylpiperazin-1-yl)benzamide;
*N*-[4-[*trans*-2-aminocyclopropyl]phenyl]-4-(1-methyl-4-piperidyl)benzamide;
*N*-[4-[*trans*-2-aminocyclopropyl]phenyl]-3-(2-oxooxazolidin-3-yl)benzamide;
*N*-[4-[*trans*-2-aminocyclopropyl]phenyl]-4-morpholino-benzamide;
*N*-[4-[*trans*-2-aminocyclopropyl]phenyl]-4-(2-oxooxazolidin-3-yl)benzamide;
benzyl *N*-[5-[[4-[(*trans*-2-aminocyclopropyl]phenyl]carbamoyl]-2-(4-methylpiperazin-1-yl)phenyl]carbamate;
benzyl *N*-[4-[[4-[*trans*-2-aminocyclopropyl]phenyl]carbamoyl]-2-(4-methylpiperazin-1-yl)phenyl]carbamate;
benzyl *N*-[5-[[4-[*trans*-2-aminocyclopropyl]phenyl]carbamoyl]-2-(1-piperidyl)phenyl]carbamate;
benzyl *N*-[5-[[4-[*trans*-2-aminocyclopropyl]phenyl]carbamoyl]-2-morpholinophenyl]carbamate
or stereoisomers or pharmaceutically acceptable salts thereof.

6. The compound according to any one of the previous claims for use as KDM1 inhibitor.

7. The compound according to any one of the previous claims for use as a medicament.

8. The compound according to any one of the previous claims for use in the treatment and/or prevention of cancer, infectious diseases or a disease **characterized by** aberration of cellular energy metabolism, such as obesity.

9. The compound according to any one of the previous claims for use in the treatment and/or prevention of leukemia, non-small cell lung cancer, hepatocellular carcinoma, or glioblastomas.

10. A pharmaceutical composition comprising a compound according to any one of the previous claims together with a pharmaceutically acceptable excipient and/or diluent.

11. The pharmaceutical composition according to claim 10 further comprising at least one therapeutic agent, preferably selected from the group consisting of histone deacetylase inhibitors, retinoid receptor modulators, anti proliferative/antineoplastic agents, cytostatic agents, agents which inhibit cancer cell invasion, inhibitors of growth factor function, antiangiogenic agents, cell cycle inhibitors, proteasome inhibitors, HSP90 inhibitors, Selective COX-2 inhibitors or a chemotherapeutic agent.

12. The pharmaceutical composition according to claim 10 or 11 in the form of tablets, capsules, oral preparations, powders, granules, pills, injectable or infusible liquid, solutions, suspensions, emulsions, suppositories, ointments, creams, lotions, gels, pastes, transdermal delivery devices.

13. A process for obtaining a compound of formula (I) according to claims 1 to 5, the process comprising the preparation of compounds of formula **A2** by reaction of a compound of formula **A1** with the a suitable azide and in presence of a base, the reaction of a compound of formula **A2** with an amide **A3** and CuI in presence of a base to obtain a compound of formula **A4,** and the deprotection of a compound of formula **A4** to obtain a compound of formula **(I),** as represented in **Scheme A** below: 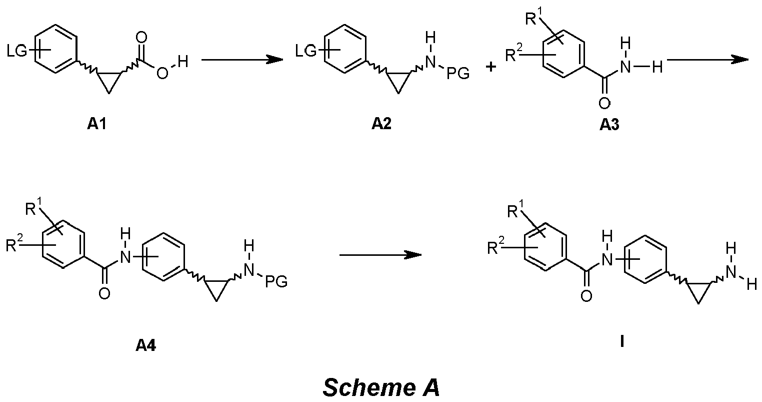 wherein **R¹** and **R²** are as defined in claim 1, **PG** is a protecting group, and **LG** is a leaving group.

14. The process according to claim 13, wherein **PG** is carboxybenzyl, *tert-*butyloxycarbonyl (BOC), or 9-fluorenylmethyloxycarbonyl.

15. Process for obtaining a compound of formula **(I)** according to claims 1 to 5, the process comprising the preparation of compounds of formula **B3** by reaction of a compound of formula **B1** with compound of formula **B2** and in presence of a condensating agent, and the deprotection of a compound of formula **B3** to obtain a compound of formula **(I),** as represented in **Scheme B** below: 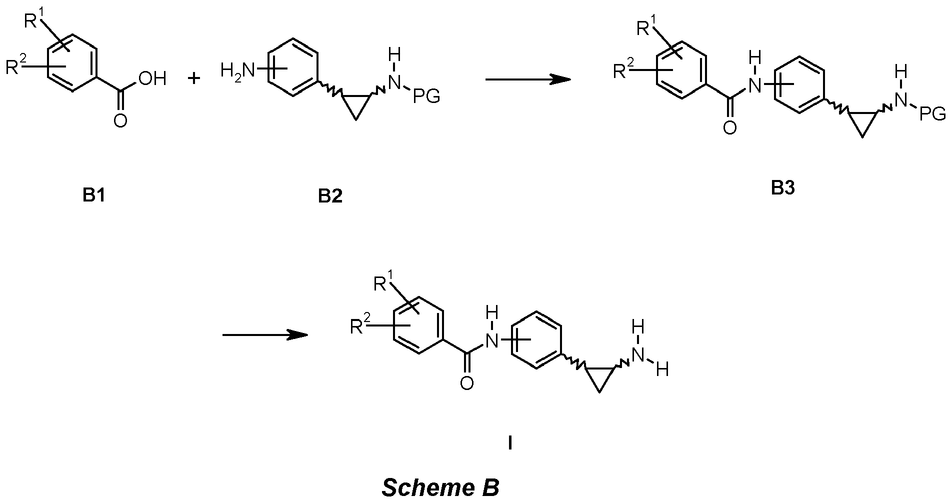 wherein **R¹** and **R²** are as defined in claim 1, **PG** is as defined in claim 13 or 14.
